# EUROPEAN PATENT SPECIFICATION

(11) **EP 2 512 411 B1**
(45) Date of publication and mention of the grant of the patent: **01.11.2017**
(21) Application number: 09801292.5
(22) Date of filing: 15.12.2009
(51) Int. Cl.: A61K 8/34, A61K 8/44, A61K 8/63, A61Q 19/00

(54) **COMPOSITION WITH ENHANCED HYDRATION ABILITY**
ZUSAMMENSETZUNG MIT ERHÖHTER HYDRIERFÄHIGKEIT
COMPOSITION À CAPACITÉ D'HYDRATATION AMÉLIORÉE

(43) Date of publication of application: 24.10.2012
(73) Proprietor: L'Oréal, 75008 Paris (FR)
(72) Inventor: JIMBO, Kazuko, Kawasaki-shi Kanagawa 213-0012 (JP); TAKANUKI, Mayumi, Kawasaki-shi Kanagawa 213-0012 (JP)
(74) Representative: Dossmann, Gérard
(86) International application number: PCT/JP2009/071196
(87) International publication number: WO 2011/074127

(56) References cited:
- EP-A1- 0 538 764
- EP-A2- 0 928 608
- EP-A2- 1 044 676
- US-A1- 2008 280 865

## Description

### TECHNICAL FIELD

The present invention relates to a cosmetic and/or dermatological composition with enhanced hydration ability which is preferably used for moisturizing the skin and/or lips.

### BACKGROUND ART

The stratum corneum is the thin cornified layer which forms the interface between the organism and the dehydrating external environment. It is formed from the upper epithelial tissue: the epidermis, and from the connected tissue located below: the dermis.

The stratum corneum, which is approximately 10 to 15 µm thick, is composed of vertically stacked corneocytes surrounded by a matrix formed from lipids organized in a lamellar liquid crystal phase. Thus, it is two- compartment system which can be compared to a brick wall, composed of anucleating cells (the "bricks") and intercellular lamellar liquid crystal phases (the "cement").

The function of the stratum corneum is in particular to regulate flows of water which enter and leave the skin and therefore to delay the excessive loss of water originating from the deeper layers of the epidermis. The stratum corneum also protects against mechanical attack and the passage of chemical products and foreign microorganisms. It also constitutes the first line of defence against UV radiation.

In general, a moisturizer or hydrating agent is defined as a substance which reduces the impression of dry skin by influencing the water content of the skin by addition or retention.

Everyone knows that it is important to keep the skin well hydrated, and notably when the skin is affected by dryness. Cosmetic and/or dermatological compositions for moisturizing the skin, in particular the dry skin, have been widely used.

For example, JP-A-H05-112514 discloses a sterol ester of an N-long chain acyl neutral amino acid having a specific chemical structure to be used as a raw material for cosmetic or dermatological compositions. According to JP-A-H05-112514, the sterol ester can enhance hydration ability and provide emollient effects.

Further, JP-A-2000-355531 discloses, as a raw material for cosmetic or dermatological compositions, a mixture of the above sterol ester and an alkyl or alkenyl ester of an N-long chain acyl neutral amino acid having a specific chemical structure. According to JP-A-2000-355531, a composition comprising the above mixture can provide good moisturizing effects such as good moisture-retaining and good moisture permeating properties.

Furthermore, JP-A-2008-260707 discloses a water-in-oil (W/O) emulsion, which is used for cosmetics, including the above mixture, silicone oil and water.

Also, glycerin is known as hydrating agent in cosmetic compositions.

### DISCLOSURE OF INVENTION

However, there is still a need for providing compositions that have enhanced moisturizing ability.

An objective of the present invention is to provide good moisturizing effects to an emulsion other than a W/O emulsion, such as an oil-in-water emulsion, which is to be used for cosmetic and dermatological compositions, including the above raw material.

The above objective of the present invention can be achieved by combining, in an oil-in-water emulsion, the above raw material with polyalcohol having 3 or more hydroxy groups.

Thus, one aspect of the present invention is a cosmetic and/or dermatological composition in the form of an emulsion other than a water-in-oil type, preferably an oil-in-water emulsion, comprising
(A) at least one N-long chain acyl neutral amino acid ester represented by formula (I): 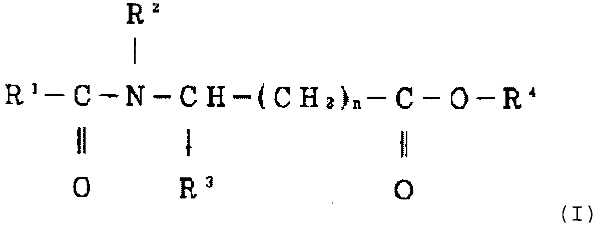 wherein
   R¹ represents a substituted or non-substituted, branched or straight alkyl or alkenyl group having 5 to 21 carbon atoms; R² and R³ independently represent a hydrogen atom or a substituted or non-substituted, branched or straight alkyl group having 1 to 4 carbon atoms;
   R⁴ represents a substituted or non-substituted, branched or straight alkyl group having 23 to 34 carbon atoms except for a behenyl group, a substituted or non-substituted, branched or straight alkenyl group having 2 to 34 carbon atoms, or a substituted or non-substituted steryl group; and
   n represents an integer of 0 to 2, and
(B) at least one polyalcohol having 3 or more hydroxy groups,
wherein
the ratio of the amount of the (B) polyalcohol having 3 or more hydroxy groups to the amount of the (A) N-long chain acyl neutral amino acid ester is 2 or more. It is preferable that R¹ in the formula (I) represent a non-substituted linear alkyl group having 10 to 21 carbon atoms; R⁴ in the formula (I) represent a non-substituted branched alkyl group having 23 to 34 carbon atoms or a phytosteryl group; and n in the formula (I) represent 1.

It is more preferable that the (A) N-long chain acyl neutral amino acid ester be N-myristoyl-N-methyl-β-alanine phytosteryl, N-myristoyl-N-methyl-β-alanine decyltetradecyl, or a mixture thereof. The mixture may comprise N-myristoyl-N-methyl-β-alanine phytosteryl and N-myristoyl-N-methyl-β-alanine decyltetradecyl in a ratio of 20:80 to 80:20.

On the other hand, it is preferable that the (B) polyalcohol having 3 or more hydroxy groups be chosen from glycerin, diglycerin, polyglycerin, or a mixture thereof.

According to the present invention, the oil phase in the composition preferably comprises at least one non-silicone oil.

In the composition according to the present invention, it is preferable that the amount of the (A) N-long chain acyl neutral amino acid ester be 0.01 to 20 wt%, relative to the total weight of the composition, and that the amount of the (B) polyalcohol having 3 or more hydroxy groups be 0.1 to 30 wt%, relative to the total weight of the composition.

It is more preferable that the ratio of the amount of the (B) polyalcohol having 3 or more hydroxy groups to the amount of the (A) N-long chain acyl neutral amino acid ester be 1 or more, preferably 2.

Advantageously, the composition according to the present invention is an oil-in-water emulsion.

The composition according to the present invention can preferably be used for hydrating the skin.

Another aspect of the present invention is a cosmetic method for treating dry skin and/or lips comprising topically applying the composition according to the present invention to the dry skin and/or lips, and a method for hydrating skin and/or lips comprising topically applying the composition according to the present invention to the skin and/or lips.

### BEST MODE FOR CARRYING OUT THE INVENTION

After diligent research, the inventors have discovered that it is possible to provide good moisturizing effects to an emulsion other than a W/O emulsion, such as an oil-in-water emulsion, which is to be used for cosmetic and dermatological compositions, including a sterol ester of an N-long chain acyl neutral amino acid preferably with an alkyl or alkenyl ester of an N-long chain acyl neutral amino acid, by combining one or more polyalcohol having 3 or more hydroxy groups with the ester(s).

Thus, the cosmetic and dermatological compositions according to the present invention are characterized by comprising:
(A) at least one N-long chain acyl neutral amino acid ester represented by formula (I): 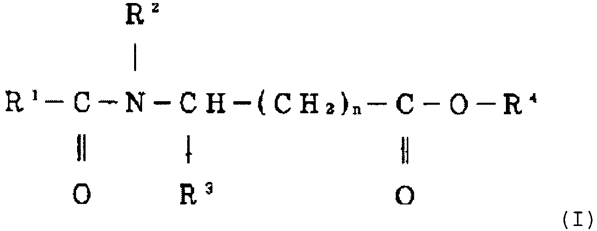 wherein
   R¹ represents a substituted or non-substituted, branched or straight alkyl or alkenyl group having 5 to 21 carbon atoms; R² and R³ independently represent a hydrogen atom or a substituted or non-substituted, branched or straight alkyl group having 1 to 4 carbon atoms;
   R⁴ represents a substituted or non-substituted, branched or straight alkyl group having 23 to 34 carbon atoms except for a behenyl group, a substituted or non-substituted, branched or straight alkenyl group having 2 to 34 carbon atoms, or a substituted or non-substituted steryl group; and
   n represents an integer of 0 to 2; and
(B) at least one polyalcohol having 3 or more hydroxy groups,
wherein
the ratio of the amount of the (B) polyalcohol having 3 or more hydroxy groups to the amount of the (A) N-long chain acyl neutral amino acid ester is 2 or more. Hereinafter, the cosmetic and/or dermatological composition according to the present invention will be explained in a more detailed manner.

### (A) N-long chain acyl neutral amino acid ester

The N-long chain acyl neutral amino acid ester used in the present invention is represented by the following chemical formula: 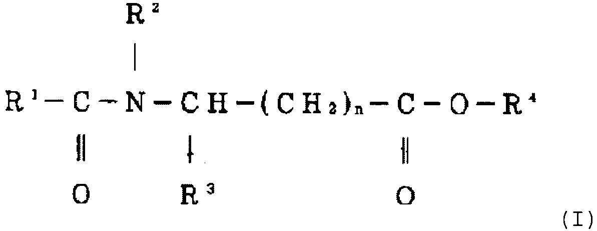 wherein
R¹ represents a substituted or non-substituted, branched or straight alkyl or alkenyl group having 5 to 21 carbon atoms;
R² and R³ independently represent a hydrogen atom or a substituted or non-substituted, branched or straight alkyl group having 1 to 4 carbon atoms;
R⁴ represents a substituted or non-substituted, branched or straight alkyl group having 23 to 34 carbon atoms except for a behenyl group, a substituted or non-substituted, branched or straight alkenyl group having 2 to 34 carbon atoms, or a substituted or non-substituted steryl group; and
n represents an integer of 0 to 2.

The long chain acyl group (R¹CO) appearing in the above-mentioned formula (I) can be a substituted or non-substituted, straight-chain or branched-chain, saturated or unsaturated one having 6 to 22 carbon atoms. As examples thereof, mention may be made of acyl groups which can be derived from capric acid, lauric acid, myristic acid, palmitic acid, stearic acid, behenic acid, linoleic acid, linolenic acid, oleic acid, isostearic acid, 2-ethylhexanoic acid, coconut oil fatty acid, palm kernel oil fatty acid, hydrogenated palm kernel oil fatty acid, beef tallow fatty acid and hydrogenated beef tallow fatty acid. Such fatty acids can be those which have a hydroxyl group. Preferred acyl groups can be caproyl group, lauroyl group, myristoyl group, palmitoyl group, stearoyl group and behenoyl group, and monovalent acid residues (acyl groups) which can be derived from coconut oil fatty acid, palm kernel oil fatty acid, hydrogenated palm kernel oil fatty acid and hydrogenated beef tallow fatty acid.

Thus, R¹ is a substituted or non-substituted, branched or straight alkyl or alkenyl group having 5 to 21 carbon atoms, preferably 7 to 21 carbon atoms, and more preferably 10 to 21 carbon atoms. It is more preferable that R¹ in the formula (I) represents a non-substituted linear alkyl group having 10 to 21 carbon atoms. In particular, a myristyl group is preferable as R¹ in the formula (I). As shown in the above-mentioned formula (I), R² and R³ can independently represent a hydrogen atom or a substituted or non-substituted, branched or straight alkyl group having 1 to 4 carbon atoms. As the alkyl group, a methyl or ethyl group is preferable. Accordingly, as the neutral amino acids constituting the neutral amino acid moieties of the N-long chain acyl neutral amino acid esters represented by the above-mentioned formula (I), mention may be made of a neutral amino acid such as glycine, alanine, valine, leucine, isoleucine, serine, threonine, proline, hydroxyproline, β-alanine, aminobutyric acid, aminocaproic acid, sarcosine or N-methyl-β-alanine. Among them, preferred ones are glycine, alanine, β-alanine, α-aminobutyric acid, γ-aminobutyric acid, sarcosine and N-methyl-β-alanine; more preferred ones are sarcosine, alanine, glycine and N-methyl-β-alanine; and most preferred ones are sarcosine and N-methyl-β-alanine. Thus, it is preferable that n in the above-mentioned formula (I) be 1. Those amino acids can be either in the optical active form or in the racemic form.

As shown in the above-mentioned formula (I), R⁴ represents a substituted or non-substituted, branched or straight alkyl group having 23 to 34 carbon atoms except for a behenyl group, a substituted or non-substituted, branched or straight alkenyl group having 2 to 34 carbon atoms, or a substituted or non-substituted steryl group.

Therefore, as the non-steryl hydrocarbon group (R⁴) constituting the alcohol moiety of the N-long chain acyl neutral amino acid ester represented by the above-mentioned formula (I), mention may be made of a substituted or non-substituted, branched-chain or straight-chain, alkyl group having 23 to 34 carbon atoms, or a substituted or non-substituted, branched-chain or straight-chain alkenyl group having 1 to 21 or 23 to 34 carbon atom(s), preferably a non-substituted branched-chain alkyl group having 23 to 34 carbon atoms. Among them, as preferred ones, there can be mentioned those alkyl groups which can be derived, for example, from decyltetradecanol. As the substituted or non-substituted steryl group (R⁴) constituting the alcohol moiety of the N-long chain acyl neutral amino acid ester represented by the above-mentioned formula (I), mention may be made of a steryl group derived from cholesterol, dihydrocholesterol, sitosterol, campesterol, stigmasterol, phytosterol, lanosterol and hydrogenated products thereof. Among them, preferred ones are cholesterol, sitosterol, campesterol, stigmasterol, phytosterol and lanosterol; more preferred ones are cholesterol, sitosterol, campesterol, stigmasterol and phytosterol; and most preferred ones are cholesterol and phytosterol. Thus, it is preferable that R⁴ be a phytosteryl group. They can be any of those derived from animals and plants and synthetic ones.

Accordingly, the most preferable N-long chain acyl neutral amino acid ester used in the present invention is N-myristoyl-N-methyl-β-alanine phytosteryl or N-myristoyl-N-methyl-β-alanine decyltetradecyl.

The N-long chain acyl neutral amino acid ester used in the present invention may be a mixture of esters in accordance with the above-mentioned formula (I).

It is preferable that the mixture comprise
(i) an N-long chain acyl neutral amino acid ester in which R⁴ in the formula (I) is a substituted or non-substituted, branched or straight alkyl group having 1 to 34 carbon atoms except for a behenyl group; and
(ii) an N-long chain acyl neutral amino acid ester in which R⁴ in the formula (I) is a substituted or non-substituted steryl group.

If the N-long chain acyl neutral amino acid ester represented by the above formula (I) is composed of the above (i) and (ii) esters, the (i) ester and the (ii) ester can be combined in a ratio-by-weight range of from 99:1 to 35:65, more preferably within a ratio-by-weight range of from 96:4 to 40:60 and, most preferably within a ratio-by-weight range of from 93:7 to 60:40. When the ratio of the (i) ester is more than 99:1, a sufficient moisturizing property may not be achieved, and whereas, when it is less than 35:65, stickiness may result and that is not preferred.

It is more preferable that the mixture comprise both N-myristoyl-N-methyl-β-alanine phytosteryl and N-myristoyl-N-methyl-β-alanine decyltetradecyl. In this case, the mixture may comprise N-myristoyl-N-methyl-β-alanine phytosteryl and N-myristoyl-N-methyl-β-alanine decyltetradecyl in a ratio of 20:80 to 80:20, preferably 30:70 to 70:30, more preferably 40:60 to 60:40, and further more preferably 50:50.

As a preferred example of this mixture, mention may be made to the one sold by Ajinomoto under the trade name ELDEW® APS-307.

The N-long chain acyl neutral amino acid ester can, for example, be prepared by condensation esterification of an N-long chain acyl neutral amino acid with an alkyl and/or alkenyl alcohol and/or a sterol by heat-dehydration either at atmospheric pressure or in vacuo. For example, 1 mole of an N-long chain acyl neutral amino acid and 1.0 to 1.2 moles of the alcohol and/or the sterol are dissolved in a non-polar solvent such as benzene or toluene, and stirred. Then, 0.01 to 1.5 moles of an acid catalyst such as sulfuric acid, p-toluenesulfonic acid, hydrogen chloride or a strong acid ion-exchange resin is added thereto and heated with stirring at 70°C to 200°C for 1 to 10 hours. During the reaction, it is preferable to remove water produced as a by-product as much as possible to accelerate the reaction.

It is also possible to prepare the N-long chain acyl neutral amino acid ester by means of an azeotropic dehydration condensation reaction using a solvent such as toluene or by means of transesterification. The N-long chain acyl neutral amino acid which is to be used for the synthesis of such an N-long chain acyl neutral amino acid ester need not be always a single compound, but can also be a mixture of N-long chain acyl neutral amino acids having different kinds of acyl groups or neutral amino acids. Likewise, the alcohol which is to be used for the synthesis of such an N-long chain acyl neutral amino acid ester need not be always a single compound, but can also be a mixture of alcohols having different chain lengths or the like.

Of course, it is also possible that an ester of an N-long chain acyl neutral amino acid with an alkyl and/or alkenyl alcohol and an ester of an N-long chain acyl neutral amino acid with a sterol are prepared separately, followed by mixing the two kinds of esters to prepare the N-long chain acyl neutral amino acid ester.

The thus-prepared N-long chain acyl neutral amino acid ester may be used as it is, in the form of a reaction mixture per se unless that jeopardizes the effects of the present invention, for the cosmetic and/or dermatological composition according to the present invention or, if necessary or if desired, it may be purified by a known and common method used by persons skilled in the art, such as a recrystallization method or column chromatography method.

It goes without saying that an N-long chain acyl neutral amino acid or salts thereof, and an alcohol or sterol, which are to be used as starting materials for the manufacture of the N-long chain acyl neutral amino acid ester, as well as neutral amino acids and the like which may be present in the N-long chain acyl neutral amino acid or salts thereof, and fatty acids or the like which may be formed as by-products, are all usually contained in cosmetic and/or dermatological compositions, and therefore, they may be contained in the cosmetic and/or dermatological composition according to the present invention as long as they do not deteriorate the effects of the present invention.

Incidentally, the N-long chain acyl neutral amino acid can be manufactured by a known method such as the so-called Schotten-Baumann reaction where a long chain fatty acid halide and an amino acid are allowed to react with each other in the presence of a basic catalyst (refer, for example, to JP-B-S51-38681).

The amount of the N-long chain acyl neutral amino acid ester is not limited. It is preferable that the amount of the N-long chain acyl neutral amino acid ester be 0.01 to 20 wt%, preferably 0.1 to 10 wt%, more preferably 0.5 to 7 wt%, and further more preferably 1 to 5 wt%, relative to the total weight of the composition.

### (B) Polyalcohol having 3 or more hydroxy groups

The polyalcohol used in the present invention as an essential element must have 3 or more hydroxy groups in a molecule.

As long as the polyalcohol has 3 or more hydroxy groups, the type of the polyalcohol is not limited. As examples of the polyalcohol having 3 or more hydroxyl groups, mention may be made of glycerols such as glycerin, diglycerin, and polyglycerin; polysaccharides such as glucose, fructose, maltose, lactose, sucrose, erythritol, xylitol, sorbitol and mannitol; gluconic acid and polyvinylalcohol. It is preferable to use polyalcohol having 3 or more hydroxy groups with a low molecular weight such as that including 3 to 100 carbon atoms, preferably 3 to 50 carbon atoms. Therefore, glycerols such as glycerin, diglycerin, and polyglycerin are preferable, and glycerin is most preferable.

The amount of the polyalcohol having 3 or more hydroxy groups is not limited. It is preferable that the amount of the polyalcohol having 3 or more hydroxy groups be 0.1 to 30 wt%, preferably 0.5 to 20 wt%, and more preferably 1 to 15 wt%, relative to the total weight of the composition.

The ratio of the amount of the polyalcohol having 3 or more hydroxy groups to the amount of the N-long chain acyl neutral amino acid ester is 2 or more.

The polyalcohol having 3 or more hydroxy groups and the N-long chain acyl neutral amino acid ester can exhibit synergetic effects in terms of hydration property for the skin, in particular dry skin. In other words, the polyalcohol having 3 or more hydroxy groups can enhance the hydration ability of the N-long chain acyl neutral amino acid ester beyond expectations based on the sum of each hydration ability of the polyalcohol having 3 or more hydroxy groups and the N-long chain acyl neutral amino acid ester. On the other hand, the N-long chain acyl neutral amino acid ester can also enhance the hydration ability of the polyalcohol having 3 or more hydroxy groups beyond expectations based on the sum of each hydration ability of the polyalcohol having 3 or more hydroxyl groups and the N-long chain acyl neutral amino acid ester.

Thus, the polyalcohol having 3 or more hydroxy groups can function as an enhancer of the hydration ability of the N-long chain acyl neutral amino acid ester, and on the other hand, the N-long chain acyl neutral amino acid ester can be used as an enhancer of the hydration ability of the polyalcohol having 3 or more hydroxy groups. Namely, the polyalcohol having 3 or more hydroxy groups can be used for enhancing the hydration ability of the N-long chain acyl neutral amino acid ester, and on the other hand, the N-long chain acyl neutral amino acid ester can be used for enhancing the hydration ability of the polyalcohol having 3 or more hydroxy groups.

Accordingly, a combination of the N-long chain acyl neutral amino acid ester and the polyalcohol having 3 or more hydroxy groups can be used in combination as an agent for hydration with synergetically enhanced hydration ability. Preferably, the above combination can be used as a hydrating agent, for the skin, in particular dry skin.

### (C) Cosmetic and/or Dermatological Composition

The cosmetic and/or dermatological composition according to the present invention includes, as essential elements, the N-long chain acyl neutral amino acid ester and the polyalcohol having 3 or more hydroxy groups. The cosmetic and/or dermatological composition according to the present invention has synergetic moisturizing effects based on the combination of the N-long chain acyl neutral amino acid ester and the polyalcohol having 3 or more hydroxy groups.

Preferably, the composition according to the present invention comprises a physiologically acceptable medium. The term "physiologically acceptable medium" is intended to mean a nontoxic medium that can be applied to the skin, the lips, the hair, the eyelashes, the eyebrows and the nails.

The composition according to the present invention is in the form of an emulsion other than the water-in-oil type, such as O/W, W/O/W and O/W/O, preferably in the form of an oil-in-water emulsion. This means that the composition according to the present invention does not comprise an oil continuous phase and aqueous phases dispersed in the oil phase, but preferably comprises an aqueous continuous phase and oil phases dispersed in the aqueous phase. Hereinafter, a preferable embodiment of the composition according to the present invention which is in the form of an oil-in-water emulsion is described.

The mean size of the droplets of the oil phase can be measured by light diffraction using a Mastersizer 2000 particle sizer (sold by Malvern Instruments). These measurements can be carried out on the emulsion diluted in a solution of SDS (sodium dodecyl sulphate) at 1% in water. A computer program makes it possible to obtain the mean diameter by volume D[4.3](µm) (see operators guide, Malvern Instruments, December 1998, p. 61 to 67).

The mean size D[4.3](µm) of the droplets of the oil phase of the composition according to the present invention may range from 0.01 µm to 6 µm, more particularly from 0.1 µm to 4 µm, and preferably from 0.1 µm to 2 µm.

The amount of the oil phase is not limited, and it can be present in an amount of 1 to 80 wt% relative to the total weight of the composition. The amount of the oil phase can range, for example, from 5 to 70 wt%, preferably from 8 to 60 wt%, more preferably 10 to 50 wt% relative to the total weight of the composition.

The oil phase may comprise only oil(s) (liquid fatty substances) or it may comprise a mixture of oils and of other fatty substances such as higher alcohols and fatty acids. However, the amount of oils is preferably at least 20 wt% relative to the total weight of the oil phase, and preferably 30 wt% or more relative to the total weight of the oil phase. The term "oil" is intended to mean a fatty substance that is liquid at ambient temperature (25°C).

The oil phase can comprise at least one non-silicone oil.

As the non-silicone oil, for example, mineral or synthetic hydrocarbon-based oil can be used. The term "hydrocarbon-based oil" is intended to mean an oil containing mainly carbon atoms and hydrogen, and optionally ester, ether and/or fluorinated groups.

As the hydrocarbon-based oils, mention may in particular be made of fatty acid esters, preferably those obtained from an alcohol comprising a saturated or unsaturated, linear or branched chain having from 1 to 30 carbon atoms and from a fatty acid comprising a linear or branched chain having from 3 to 30 carbon atoms, the total sum of carbon atoms of said esters being greater than or equal to 24; alkanes; and mixtures thereof.

As fatty acid esters, mention may, for example, be made of 2-ethylhexyl palmitate (or octyl palmitate), cetyl 2-ethylhexanoate, 2-octyldodecyl myristate, isocetyl stearate, isostearyl isostearate, 2-ethylhexyl stearate (or octyl stearate), octyldodecyl neopentanoate, cetearyl isononanoate, isodecyl isononanoate, pentaerythritol tetraisostearate, isotridecyl isononanoate, C₁₂-C₁₅ fatty alcohol benzoates (Finsolv TN from Finetex), and mixtures thereof.

The amount of the hydrocarbon-based oil is not limited, however, it is preferable that it is at least 10% of the total weight of the oil phase, and preferably at least 20% of the total weight of the oil phase. This amount preferably ranges from 10 to 100 wt%, and better still from 20 to 90 wt% of the total weight of the oil phase.

As alkanes, mention may, for example, be made of petroleum jelly, liquid petroleum jelly, hydrogenated isoparaffins such as Parleam® oil sold by the company NOF Corporation (INCI name: Hydrogenated Polyisobutene), and mixtures thereof.

According to a more preferred embodiment of the present invention, the composition according to the present invention contains squalane.

The oily phase can comprise other oil(s). As the other oil(s), mention may in particular be made of esters of molecular weight less than 360 g/mol, such as 2-ethylhexyl caprate/caprylate (or octyl caprate/caprylate), ethyl laurate, butyl laurate, hexyl laurate, isohexyl laurate, isopropyl laurate, methyl myristate, ethyl myristate, butyl myristate, isobutyl myristate, isopropyl myristate, 2-ethylhexyl monococoate (or octyl monococoate), methyl palmitate, ethyl palmitate, isopropyl palmitate, isobutyl palmitate, butyl stearate, isopropyl stearate, isobutyl stearate, isopropyl isostearate, 2-ethylhexyl pelargonate (or octyl pelargonate) 2-ethylhexyl hydroxystearate (or octyl hydroxystearate), decyl oleate, diisopropyl adipate, 2-diethylhexyl adipate (or dioctyl adipate), diisocetyl adipate, 2-ethylhexyl succinate (or octyl succinate), diisopropyl sebacate, 2-ethylhexyl malate (or octyl malate), pentaerythritol caprate/caprylate,2-ethylhexyl hexanoate, (or octyl hexanoate), octyldodecyl octanoate, isodecyl neopentanoate, isostearyl neopentanoate, isononyl isononanoate, isotridecyl isononanoate, lauryl lactate, myristyl lactate, cetyl lactate, myristyl propionate, 2-ethylhexyl 2-ethylhexanoate (or octyl 2-ethylhexanoate), 2-ethylhexyl octanoate (or octyl octanoate), and mixtures thereof; isopropyl lauroyl sarcosinate (Eldew SL 205 from Unipex), dicaprylyl carbonate (Cetiol CC from Cognis); ethers such as dicaprylyl ether (Cetiol OE from Cognis); hydrocarbon-based oils of plant origin, such as sweet almond oil, avocado oil, castor oil, coriander oil, olive oil, jojoba oil, sesame oil, groundnut oil, grapeseed oil, rape seed oil, coconut oil, hazelnut oil, shea butter, palm oil, apricot kernel oil, beauty-leaf oil, rice bran oil, corn germ oil, wheat germ oil, soybean oil, sunflower oil, evening primrose oil, safflower oil, passion flower oil, rye oil, caprylic/capric acid triglycerides such as those sold by the company Stearineries Dubois or those sold under the names Miglyol 810, 812 and 818 by the company Dynamit Nobel;
volatile or non-volatile silicone oils, such as volatile or non-volatile polydimethylsiloxanes (PDMS) containing a linear or cyclic silicone chain, that are liquid or pasty at ambient temperature, in particular cyclopolydimethylsiloxanes (cyclomethicones) such as cyclohexasiloxane; polydimethylsiloxanes containing alkyl, alkoxy or phenyl groups that are pendent or at the end of the silicone chain, which groups have from 2 to 24 carbon atoms; phenyl silicones such as phenyl trimethicones, phenyl dimethicones, phenyltrimethyl-siloxydiphenylsiloxanes, diphenyl dimethicones, diphenylmethyldiphenyltrisiloxanes, 2-phenylethyltrimethyl siloxysilicates, polymethylphenylsiloxanes; [0054] fluoro oils such as those that are partially hydrocarbon-based and/or silicone based, for instance those described in document JP-A-2-295912;
linear or branched hydrocarbon-based oils of mineral, synthetic or animal origin, chosen from isohexadecane, isododecane, C₈₋₉ and C₁₁₋₁₃ isoparaffins (CTFA name: C₈₉ Isoparaffin and C₁₁₋₁₃ Isoparaffin) ; and mixtures thereof.

The composition according to the present invention may comprise an amount of an aqueous phase less than or equal to 99 wt%, preferably less than or equal to 90 wt% of the total weight of the composition, it being possible for this amount to range, for example, from 20 to 99 wt%, and preferably from 30 to 90% by weight, relative to the total weight of the composition. The amount of water in the aqueous phase can range, for example, from 50 to 100% by weight relative to the weight of the aqueous phase.

According to a preferred embodiment, the composition according to the present invention may contain at least one polyol (or polyhydric alcohol) which has two hydroxy groups that is generally present in the aqueous phase as long as it does not impair the effects of the present invention. As such polyols, mention may, for example, be made of glycols such as propylene glycol, butylene glycol, isoprene glycol and polyethylene glycols such as PEG-8. The amount of such polyol generally ranges from 0.1 to 30 wt%, and better still from 0.5 to 20 wt%, relative to the total weight of the aqueous phase.

The aqueous phase can contain, for example, besides the water and the above optional polyol(s) having two hydroxy groups, one or more water-soluble solvents chosen from water-soluble lower alcohol(s). The term "lower alcohol" is intended to mean an alcohol containing from 1 to 8 carbon atoms. As lower alcohols, mention may, for example, be made of ethanol, isopropanol, butanol and mixtures thereof. When they are present in the composition according to the present invention, the water-soluble lower alcohol(s) may be in an amount ranging from 0.01 to 40 wt%, and preferably from 0.01 to 20 wt%, relative to the total weight of the aqueous phase.

The composition according to the present invention may also contain any adjuvant or additive, for example those conventionally used in the fields under consideration, and in particular in the cosmetics or dermatological fields. Of course, those skilled in the art will take care to choose the optional additive(s) of the composition according to the present invention in such a way that the advantageous properties intrinsically associated with the composition in accordance with the present invention are not, or are not substantially, altered by the envisaged addition.

Among the conventional adjuvants that may be contained in the aqueous phase and/or in the oil phase of the emulsions in accordance with the present invention (according to the water-soluble or liposoluble nature of these adjuvants), mention may in particular be made of foaming surfactants that are anionic (such as sodium lauryl ether sulphate, sodium alkyl phosphate or sodium trideceth sulphate), amphoteric (such as alkyl betaine or disodium cocoamphodiacetate) or non-ionic with an HLB greater than 10 (such as POE/PPG/POE, alkylpolyglucoside, or polyglyceryl-3 hydroxylauryl ether); preserving agents; sequestering agents (EDTA); antioxidants; fragrances; dyestuffs such as soluble dyes, pigments and pearlescent agents; matifying, tensioning, bleaching or exfoliating fillers; sunscreens; cosmetic or dermatological active agents and agents having the effect of improving the cosmetic properties of the skin, that are hydrophilic or lipophilic; electrolytes; hydrophilic or lipophilic, anionic, non-ionic, cationic or amphoteric, thickening or dispersing polymers. The amounts of these various adjuvants are those conventionally used in the field under consideration and are, for example, from 0.01 to 20 wt% of the total weight of the composition.

As active agents that can be used in the composition according to the present invention, mention may, for example, be made of water-soluble or liposoluble vitamins such as vitamin A (retinol), vitamin E (tocopherol), vitamin C (ascorbic acid), vitamin B5 (panthenol) vitamin B3 (niacinamide), derivatives of these vitamins (in particular esters) and mixtures thereof; antiseptics; antibacterial active agents such as 2,4,4'-trichloro-2'-hydroxy diphenyl ether (or triclosan) or 3,4,4'-trichlorocarbanilide (or triclocarban); antisebohrreic agents; antimicrobial agents such as benzyl peroxide, salicylic acid, triclosan, azelaic acid or niacin (vit. PP); slimming agents such as caffeine; optical brighteners, and any active agent that is suitable for the final purpose of the composition, and mixtures thereof.

The amount of active agents depends on the desired aim. The active agent(s) may, for example, be present at a concentration ranging from 0.001 to 20 wt%, preferably from 0.01 to 10 wt%, and better still from 0.05 to 5 wt% relative to the total weight of the composition.

The composition according to the present invention typically comprises an emulsifier or emulsifiers to disperse the oil phases in the aqueous phase. The type of the emulsifier(s) is not limited. As examples of the emulsifier(s), mention may be made of fatty acid esters of polyethyleneglycols (PEG-100 stearate or the like) and fatty acid esters of glycerols (Glyceryl stearate or the like).
The mixture of Glyceryl Stearate and PEG-100 Stearate is preferred as emulsifier. The amount of the emulsifier(s) is not limited, but it may be 0.001 to 15 wt% relative to the total weight of the composition.

The emulsion according to the present invention can be obtained by a conventional process such as adding the materials for the oily phases to the materials for the aqueous phase followed by stirring.

Alternatively, the emulsion according to the present invention may be prepared by a phase inversion process. This preparation process may comprise:
1) Weighing out, into a container, all the constituents of the composition (with the exception of thermosensitive starting materials, if there are any).
2) Homogenizing the mixture, for example by means of a Rayneri 350 rpm, and heating by gradually increasing the temperature, for example by means of a water bath, to a temperature greater than or equal to the phase inversion temperature T2, i.e., until a transparent or translucent phase (microemulsion or lamellar phase region) is obtained, followed by a more viscous white phase that indicates that the inverse emulsion (W/O) has been obtained.
3) Stopping the heating and maintaining the stirring until ambient temperature is again reached, passing through the phase inversion temperature T1, i.e. the temperature at which a fine O/W emulsion is obtained.
4) When the temperature has again dropped below the phase inversion temperature (T1) region, optionally adding the thermosensitive starting materials.

The composition according to the present invention is in the form of an emulsion other than the water-in-oil type, such as O/W, W/O/W and O/W/O, preferably in the form of an O/W emulsion, and it can in particular constitute a cosmetic or dermatological composition, for example cosmetic compositions for the treatment of keratin materials such as the skin, in particular the face, the mucous membranes, the eyelashes, the scalp, the hair and the nails. The emulsion according to the present invention may be such as a cream, lotion, gel, impregnated towels, patch, washing product such as body wash, face wash.
They can also constitute, for example, makeup-removing compositions and/or cleansing compositions and/or care compositions for the skin, the mucous membranes such as the lips, and/or for the eyelashes, compositions for massaging facial skin or body skin, scrubbing (or exfoliating) compositions both for the face and for the hands (when the composition contains exfoliating particles), antisun compositions (UV protection) and aftersun compositions. They may also constitute make-up compositions for keratin materials and in particular the skin, the lips and the eyelashes, and more particularly such as foundations, lipsticks or lip glosses after the addition of suitable pigments and/or fillers.

The compositions according to the present invention can also be used as shower care balms (to be rinsed, for example by massaging in the product until the oil is released, and then rinsing the skin which is then soft and moisturized); as conditioners and hair care balms; as shaving products; as masks, including an aftersun repairing mask; as a slimming poultice on a region of "orange peel skin" (to be massaged in and then rinsed off); as a massage balm; as a lip repairing balm to be rinsed off; as a balm for dry feet. In these uses, the product is subsequently rinsed off.

When the composition is an exfoliating product (also called cleansing product), the use comprises applying the product to the face or the hands or the body, in rubbing for one or two minutes, and then in rinsing. The skin is then smooth, soft and cleansed.

The composition according to the present invention is preferably used for hydrating the skin, in particular the face. Preferably, the composition according to the present invention can be used for treating dry skin.

Therefore, the composition according to the present invention can be used for a method for treating dry skin and/or lips, preferably cosmetically treating dry skin and/or lips, which comprises topically applying to said dry skin and/or lips, the composition according to the present invention.

Alternatively, the composition according to the present invention can be used for a method for hydrating skin and/or lips, in particular dry skin and/or lips, which comprises topically applying to said skin and/or lips, the composition according to the present invention.

Alternatively, the composition according to the present invention can be used for a method for hydrating skin and/or lips in need, in particular human skin and/or lips in need. By human skin it is understood any human skin such as adult, child or baby skin.

### EXAMPLES

The present invention will be described in more detail by way of examples, which however should not be construed as limiting the scope of the present invention.

In the following examples, the Phytosteryl/Decyltetradecyl Myristoyl methyl β-alanine used is the one sold by Ajinomoto under the trade name ELDEW® APS-307.

The Corneometer® used (CM 825, Courage + Khazaka, Germany) quantified moisture content in the stratum corneum (SC) by an electrical capacitance method. This measurement uses arbitrary units that increase as the skin becomes more hydrated.

### Example 1 and Comparative Examples 1 to 3

The following compositions in the form of O/W emulsions according to Example 1 and Comparative Examples 1 to 3 were prepared by mixing the components shown in Table 1 in accordance with the preparation process described below. The numerals in Table 1 mean percent by weight, relative to the total weight of the composition.

**Table 1**

| | Ingredient | Ex. 1 | Comp. Ex. 1 | Comp. Ex. 2 | Comp. Ex. 3 |
|---|---|---|---|---|---|
| A | Water | 58.27 | 68.27 | 65.27 | 61.27 |
| | Glycerin | 7.00 | - | - | 7.00 |
| | Methylparaben | 0.30 | 0.30 | 0.30 | 0.30 |
| | Tetrasodium EDTA | 0.20 | 0.20 | 0.20 | 0.20 |
| | Phenoxyethanol | 0.50 | 0.50 | 0.50 | 0.50 |
| | Citric acid | 0.03 | 0.03 | 0.03 | 0.03 |
| B | Glyceryl Stearate PEG-100 Stearate | 3.00 | 3.00 | 3.00 | 3.00 |
| | Stearic acid | 0.60 | 0.60 | 0.60 | 0.60 |
| | Cetyl alcohol | 0.40 | 0.40 | 0.40 | 0.40 |
| | Squalane | 4.00 | 4.00 | 4.00 | 4.00 |
| | Phytosteryl/Decyltetradecyl Myristoyl methyl β-alanine | 3.00 | - | 3.00 | - |
| C | Phenyl trimethicone | 2.00 | 2.00 | 2.00 | 2.00 |
| | Carbomer | 0.30 | 0.30 | 0.30 | 0.30 |
| D | Xanthan gum | 0.10 | 0.10 | 0.10 | 0.10 |
| | Water | 10.00 | 10.00 | 10.00 | 10.00 |
| E | Triethanolamine | 0.30 | 0.30 | 0.30 | 0.30 |
| | Water | 10.00 | 10.00 | 10.00 | 10.00 |

### Preparation Process:

1) Phases A and B were each heated up to about 70-75°C, separately.
2) Phase B was poured into Phase A.
3) The mixture was homogenized with a homogenizer at 8000 rpm for 5 minutes.
4) Phase C was added to the mixture and stirred.
5) The mixture was cooled, and Phases D and E were added.

For Example 1 and Comparative Examples 1-3, the hydration property was measured and evaluated in accordance with the following process.

### Test Process:

1) For 24 female volunteers, the bare skin condition of the forearms was measured with Corneometer® (CM 825, Courage + Khazaka, Germany).
2) The composition of Example 1 and Comparative Examples 1-3 was applied in an amount of 2mg/cm² to the forearms.
3) One hour after application, the skin condition of the forearms was measured again with Corneometer® (CM 825, Courage + Khazaka, Germany).

The results of the test are shown in Table 2.

**Table 2**

| Hydration Measurement Results | Ex. 1 | Comp. Ex. 1 | Comp. Ex. 2 | Comp. Ex. 3 |
|---|---|---|---|---|
| Before Application (T0) | 34.70 | 33.64 | 34.60 | 35.78 |
| 1 Hour After Application (T1) | 54.00 | 39.96 | 40.32 | 51.37 |
| T1-T0 | 19.30 | 6.32 | 5.72 | 15.59 |
| Difference from Comp. Ex. 1 | +12.98 | 0 | -0.6 | +9.27 |

Table 2 shows that phytosteryl/decyltetradecyl myristoyl methyl β-alanine alone does not have any hydration enhancing effect.
On the other hand, Table 2 clearly shows that a combination of phytosteryl/decyltetradecyl myristoyl methyl β-alanine and glycerin exhibits a synergetic effect in terms of hydration of the skin. The sum (-0.6+9.27=8.67) of the differences (from Comparative Example 1) for Comparative Examples 2 and 3 is far below the difference (+12.98) for Example 1.

### Comparative Examples 4 to 7

The following compositions in the form of O/W emulsions according to Comparative Examples 4 to 7 were prepared by mixing the components shown in Table 3 in accordance with the preparation process described below. The numerals in Table 3 mean percent by weight, relative to the total weight of the composition.

**Table 3**

| | Ingredient | Comp. Ex. 4 | Comp. Ex. 5 | Comp. Ex. 6 | Comp. Ex. 7 |
|---|---|---|---|---|---|
| A | Water | 58.27 | 68.27 | 65.27 | 61.27 |
| | Butyleneglycol | 7.00 | - | - | 7.00 |
| | Methylparaben | 0.30 | 0.30 | 0.30 | 0.30 |
| | Tetrasodium EDTA | 0.20 | 0.20 | 0.20 | 0.20 |
| | Phenoxyethanol | 0.50 | 0.50 | 0.50 | 0.50 |
| | Citric acid | 0.03 | 0.03 | 0.03 | 0.03 |
| B | Glyceryl Stearate PEG-100 Stearate | 3.00 | 3.00 | 3.00 | 3.00 |
| | Stearic acid | 0.60 | 0.60 | 0.60 | 0.60 |
| | Cetyl alcohol | 0.40 | 0.40 | 0.40 | 0.40 |
| | Squalane | 4.00 | 4.00 | 4.00 | 4.00 |
| | Phytosteryl/Decyltetradecyl Myristoyl methyl β-alanine | 3.00 | - | 3.00 | - |
| C | Phenyl trimethicone | 2.00 | 2.00 | 2.00 | 2.00 |
| | Carbomer | 0.30 | 0.30 | 0.30 | 0.30 |
| D | Xanthan gum | 0.10 | 0.10 | 0.10 | 0.10 |
| | Water | 10.00 | 10.00 | 10.00 | 10.00 |
| E | Triethanolamine | 0.30 | 0.30 | 0.30 | 0.30 |
| | Water | 10.00 | 10.00 | 10.00 | 10.00 |

### Preparation Process:

1) Phases A and B were each heated up to about 70-75°C, separately.
2) Phase B was poured into Phase A.
3) The mixture was homogenized with a homogenizer at 8000 rpm for 5 minutes.
4) Phase C was added to the mixture and stirred.
5) The mixture was cooled, and Phases D and E were added.

For Comparative Examples 4-7, the hydration property was measured and evaluated in accordance with the following process.

### Test Process:

1) For 24 female volunteers, the bare skin condition of the forearms was measured with Corneometer® (CM 825, Courage + Khazaka, Germany)..
2) The composition of Comparative Examples 4-7 was applied in an amount of 2mg/cm² to the forearms.
3) One hour after application, the skin condition of the forearms was measured again with Corneometer® (CM 825, Courage + Khazaka, Germany).

The results of the test are shown in Table 4.

**Table 4**

| Hydration Measurement Results | Comp. Ex. 4 | Comp. Ex. 5 | Comp. Ex. 6 | Comp. Ex. 7 |
|---|---|---|---|---|
| Before Application (T0) | 29.31 | 33.64 | 34.60 | 29.24 |
| 1 Hour After Application (T1) | 34.08 | 39.96 | 40.32 | 33.76 |
| T1-T0 | 4.78 | 6.32 | 5.72 | 4.53 |
| Difference from Comp. Ex. 5 | -1.54 | 0 | -0.6 | -1.79 |

Table 4 clearly shows that a combination of
phytosteryl/decyltetradecyl myristoyl methyl β-alanine and butyleneglycol which is a compound having two hydroxy groups in a molecule exhibits no improvement in terms of hydration of the skin.

### Comparative Examples 8 to 11

The following compositions in the form of O/W emulsions according to Comparative Examples 8 to 11 were prepared by mixing the components shown in Table 5 in accordance with the preparation process described below. The numerals in Table 5 mean percent by weight, relative to the total weight of the composition.

**Table 5**

| | Ingredient | Comp. Ex. 8 | Comp. Ex. 9 | Comp. Ex. 10 | Comp. Ex. 11 |
|---|---|---|---|---|---|
| A | Water | 58.27 | 68.27 | 65.27 | 61.27 |
| | Dipropyleneglycol | 7.00 | - | - | 7.00 |
| | Methylparaben | 0.30 | 0.30 | 0.30 | 0.30 |
| | Tetrasodium EDTA | 0.20 | 0.20 | 0.20 | 0.20 |
| | Phenoxyethanol | 0.50 | 0.50 | 0.50 | 0.50 |
| | Citric acid | 0.03 | 0.03 | 0.03 | 0.03 |
| B | Glyceryl Stearate PEG-100 Stearate | 3.00 | 3.00 | 3.00 | 3.00 |
| | Stearic acid | 0.60 | 0.60 | 0.60 | 0.60 |
| | Cetyl alcohol | 0.40 | 0.40 | 0.40 | 0.40 |
| | Squalane | 4.00 | 4.00 | 4.00 | 4.00 |
| | Phytosteryl/Decyltetradecyl Myristoyl methyl β-alanine | 3.00 | - | 3.00 | - |
| C | Phenyl trimethicone | 2.00 | 2.00 | 2.00 | 2.00 |
| | Carbomer | 0.30 | 0.30 | 0.30 | 0.30 |
| D | Xanthan gum | 0.10 | 0.10 | 0.10 | 0.10 |
| | Water | 10.00 | 10.00 | 10.00 | 10.00 |
| E | Triethanolamine | 0.30 | 0.30 | 0.30 | 0.30 |
| | Water | 10.00 | 10.00 | 10.00 | 10.00 |

### Preparation Process:

1) Phases A and B were each heated up to about 70-75°C, separately.
2) Phase B was poured into Phase A.
3) The mixture was homogenized with a homogenizer at 8000 rpm for 5 minutes.
4) Phase C was added to the mixture and stirred.
5) The mixture was cooled, and Phases D and E were added.

For Comparative Examples 8-11, the hydration property was measured and evaluated in accordance with the following process.

### Test Process:

1) For 24 female volunteers, the bare skin condition of the forearms was measured with Corneometer® (CM 825, Courage + Khazaka, Germany).
2) The composition of Comparative Examples 8-11 was applied in an amount of 2mg/cm² to the forearms.
3) One hour after application, the skin condition of the forearms was measured again with Corneometer® (CM 825, Courage + Khazaka, Germany).

The results of the test are shown in Table 6.

**Table 6**

| Hydration Measurement Results | Comp. Ex. 8 | Comp. Ex. 9 | Comp. Ex. 10 | Comp. Ex. 11 |
|---|---|---|---|---|
| Before Application (T0) | 29.18 | 33.64 | 34.6 | 28.74 |
| 1 Hour After Application (T1) | 35.15 | 39.96 | 40.32 | 34.33 |
| T1-T0 | 5.97 | 6.32 | 5.72 | 5.60 |
| Difference from Comp. Ex. 9 | -0.35 | 0 | -0.6 | -0.72 |

Table 6 clearly shows that a combination of phytosteryl/decyltetradecyl myristoyl methyl β-alanine and dipropyleneglycol which is a compound having two hydroxy groups in a molecule exhibits no improvement in terms of hydration of the skin.

It should be noted that Comparative Example 1 corresponds to Comparative Examples 5 and 9, and that Comparative Example 2 corresponds to Comparative Examples 6 and 10.

## Claims

1. A cosmetic and/or dermatological composition in the form of an emulsion other than the water-in-oil type, comprising:
(A) at least one N-long chain acyl neutral amino acid ester represented by formula (I): 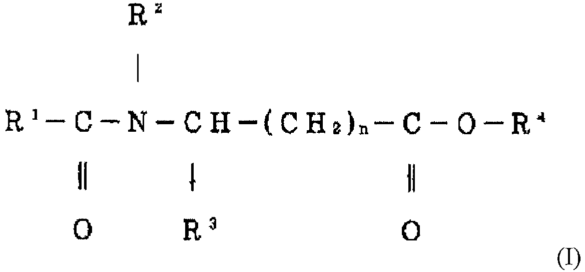 wherein:
R¹ represents a substituted or non-substituted, branched or straight alkyl or alkenyl group having 5 to 21 carbon atoms;
R² and R³ independently represent a hydrogen atom or a substituted or non-substituted, branched or straight alkyl group having 1 to 4 carbon atoms;
R⁴ represents a substituted or non-substituted, branched or straight alkyl group having 23 to 34 carbon atoms except for a behenyl group, a substituted or non-substituted, branched or straight alkenyl group having 2 to 34 carbon atoms, or a substituted or non-substituted steryl group; and
n represents an integer of 0 to 2, and
(B) at least one polyalcohol having 3 or more hydroxy groups,
wherein
the ratio of the amount of the (B) polyalcohol having 3 or more hydroxy groups to the amount of the (A) N-long chain acyl neutral amino acid ester is 2 or more.

2. The composition according to Claim 1, wherein R¹ in the formula (I) represents a non-substituted linear alkyl group having 10 to 21 carbon atoms.

3. The composition according to any one of Claims 1 to 2, wherein R⁴ in the formula (I) represents a non-substituted branched alkyl group having 23 to 34 carbon atoms or a phytosteryl group.

4. The composition according to any one of Claims 1 to 3, wherein n in the formula (I) represents 1.

5. The composition according to any one of Claims 1 to 4, wherein said (A) N-long chain acyl neutral amino acid ester is N-myristoyl-N-methyl-β-alanine phytosteryl, N-myristoyl-N-methyl-β-alanine decyltetradecyl, or a mixture thereof.

6. The composition according to Claim 5, wherein the mixture comprises N-myristoyl-N-methyl-β-alanine phytosteryl and N-myristoyl-N-methyl-β-alanine decyltetradecyl in a ratio of 20:80 to 80:20.

7. The composition according to any one of Claims 1 to 6, wherein said (B) polyalcohol having 3 or more hydroxy groups is chosen from glycerin, diglycerin, polyglycerin, or a mixture thereof.

8. The composition according to any one of Claims 1 to 7, wherein the oil phase comprises at least one non-silicone oil.

9. The composition according to any one of Claims 1 to 8, wherein the amount of said (A) N-long chain acyl neutral amino acid ester is 0.01 to 20 wt%, relative to the total weight of the composition.

10. The composition according to any one of Claims 1 to 9, wherein the amount of said (B) polyalcohol having 3 or more hydroxy groups is 0.1 to 30 wt%, relative to the total weight of the composition.

11. The composition according to any one of Claims 1 to 10, wherein the emulsion is an oil-in-water emulsion.

12. The composition according to any one of Claims 1 to 11, wherein the composition is intended for hydrating skin.

13. A cosmetic method for treating dry skin and/or lips comprising topically applying the composition according to any one of Claims 1 to 11 to said dry skin and/or lips.

14. A cosmetic method for hydrating skin and/or lips comprising topically applying the composition according to any one of Claims 1 to 11 to said skin and/or lips.

## Patentansprüche

1. Kosmetische und/oder dermatologische Zusammensetzung in Form einer Emulsion, die eine andere als vom Wasser-in-Öl-Typ ist, umfassend:
(A) wenigstens einen neutralen N-Langkettenacylaminosäureester, dargestellt durch die Formel (I): 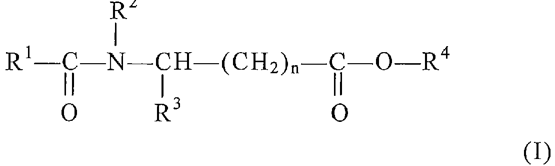 wobei:
R¹ für eine substituierte oder nicht substituierte, verzweigte oder gerade Alkyl- oder
Alkenylgruppe mit 5 bis 21 Kohlenstoffatomen steht;
R² und R³ unabhängig voneinander für ein Wasserstoffatom oder eine substituierte oder nicht substituierte, gerade oder verzweigte Alkylgruppe mit 1 bis 4 Kohlenstoffatomen stehen;
R⁴ für eine substituierte oder nicht substituierte, verzweigte oder gerade Alkylgruppe mit 23 bis 34 Kohlenstoffatomen mit Ausnahme einer Behenylgruppe, eine substituierte oder nicht substituierte, verzweigte oder gerade Alkenylgruppe mit 2 bis 34 Kohlenstoffatomen oder eine substituierte oder nicht substituierte Sterylgruppe steht; und
n für eine ganze Zahl von 0 bis 2 steht, und
(B) wenigstens einen Polyalkohol mit 3 oder mehr Hydroxygruppen,
wobei
das Verhältnis der Menge des Polyalkohols mit 3 oder mehr Hydroxygruppen (B) zu der Menge des neutralen N-Langkettenacylaminosäureesters (A) 2 oder mehr beträgt.

2. Zusammensetzung nach Anspruch 1, wobei R¹ in der Formel (1) für eine nicht substituierte lineare Alkylgruppe mit 10 bis 21 Kohlenstoffatomen steht.

3. Zusammensetzung nach einem der Ansprüche 1 bis 2, wobei R⁴ in der Formel (I) für eine nicht substituierte verzweigte Alkylgruppe mit 23 bis 34 Kohlenstoffatomen oder eine Phytosterylgruppe steht.

4. Zusammensetzung nach einem der Ansprüche 1 bis 3, wobei n in der Formel (1) für 1 steht.

5. Zusammensetzung nach einem der Ansprüche 1 bis 4, wobei es sich bei dem neutralen N-Langkettenacylaminosäureester (A) um N-Myristoyl-N-methyl-β-alaninphytosteryl, N-Myristoyl-N-methyl-β-alanin-decyltetradecyl oder eine Mischung davon handelt.

6. Zusammensetzung nach Anspruch 5, wobei die Mischung N-Myristoyl-N-methyl-β-alanin-phytosteryl und N-Myristoyl-N-methyl-β-alanin-decyltetradecyl in einem Verhältnis von 20:80 bis 80:20 umfasst.

7. Zusammensetzung nach einem der Ansprüche 1 bis 6, wobei der Polyalkohol mit 3 oder mehr Hydroxygruppen (B) aus Glycerin, Diglycerin, Polyglycerin oder einer Mischung davon ausgewählt ist.

8. Zusammensetzung nach einem der Ansprüche 1 bis 7, wobei die Ölphase wenigstens ein Nichtsilikonöl umfasst.

9. Zusammensetzung nach einem der Ansprüche 1 bis 8, wobei die Menge des neutralen N-Langkettenacylaminosäureesters (A) 0,01 bis 20 Gew.-% bezogen auf das Gesamtgewicht der Zusammensetzung beträgt.

10. Zusammensetzung nach einem der Ansprüche 1 bis 9, wobei die Menge des Polyalkohols mit 3 oder mehr Hydroxygruppen (B) 0,1 bis 30 Gew.-% bezogen auf das Gesamtgewicht der Zusammensetzung beträgt.

11. Zusammensetzung nach einem der Ansprüche 1 bis 10, wobei die Emulsion eine Ölin-Wasser-Emulsion ist.

12. Zusammensetzung nach einem der Ansprüche 1 bis 11, wobei die Zusammensetzung zum Hydratisieren von Haut bestimmt ist.

13. Kosmetisches Verfahren zur Behandlung von trockener Haut und/oder trockenen Lippen, umfassend das topische Auftragen der Zusammensetzung nach einem der Ansprüche 1 bis 11 auf die trockene Haut und/oder die trockenen Lippen.

14. Kosmetisches Verfahren zum Hydratisieren von Haut und/oder Lippen, umfassend das topische Auftragen der Zusammensetzung nach einem der Ansprüche 1 bis 11 auf die Haut und/oder die Lippen.

## Revendications

1. Composition cosmétique et/ou dermatologique, se présentant sous la forme d'une émulsion autre que de type eau-dans-huile et comprenant :
A) au moins un ester d'acide N-(acyle à longue chaîne)-aminé neutre, représenté par la formule (I) : 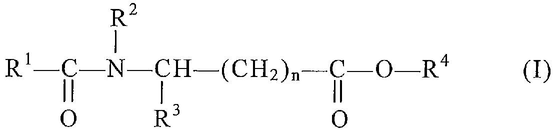 dans laquelle
- R¹ représente un groupe alkyle ou alcényle, linéaire ou ramifié, avec ou sans substituant(s) et comportant de 5 à 21 atomes de carbone,
- R² et R³ représentent, indépendamment, un atome d'hydrogène ou un groupe alkyle, linéaire ou ramifié, avec ou sans substituant(s) et comportant de 1 à 4 atomes de carbone,
- R⁴ représente un groupe alkyle, linéaire ou ramifié, avec ou sans substituant(s) et comportant de 23 à 34 atomes de carbone, excepté un groupe béhényle, ou un groupe alcényle, linéaire ou ramifié, avec ou sans substituant(s) et comportant de 2 à 34 atomes de carbone, ou un groupe stéryle, avec ou sans substituant(s),
- et l'indice n est un nombre entier valant de 0 à 2 ;
B) et au moins un polyol comportant 3 groupes hydroxyle ou plus, dans laquelle le rapport de la quantité de polyol (B) comportant trois groupes hydroxyle ou plus à la quantité d'ester (A) d'acide N-(acyle à longue chaîne)-aminé neutre vaut 2 ou plus.

2. Composition conforme à la revendication 1, dans laquelle R¹, dans la formule (I), représente un groupe alkyle linéaire sans substituant, comportant de 10 à 21 atomes de carbone.

3. Composition conforme à l'une des revendications 1 à 2, dans laquelle R⁴, dans la formule (I), représente un groupe alkyle ramifié sans substituant, comportant de 23 à 34 atomes de carbone, ou un groupe phytostéryle.

4. Composition conforme à l'une des revendications 1 à 3, dans laquelle l'indice n de la formule (I) vaut 1.

5. Composition conforme à l'une des revendications 1 à 4, dans laquelle ledit ester (A) d'acide N-(acyle à longue chaîne)-aminé neutre est de l'ester de phytostéryle de N-myristoyl-N-méthyl-β-alanine, de l'ester de décyl-tétradécyle de N-myristoyl-N-méthyl-β-alanine, ou un mélange de ceux-ci.

6. Composition conforme à la revendication 5, dans laquelle le mélange comprend de l'ester de phytostéryle de N-myristoyl-N-méthyl-β-alanine et de l'ester de décyl-tétradécyle de N-myristoyl-N-méthyl-β-alanine en un rapport de 20/80 à 80/20.

7. Composition conforme à l'une des revendications 1 à 6, dans laquelle ledit polyol (B) comportant trois groupes hydroxyle ou plus est choisi parmi du glycérol, du diglycérol, un polyglycérol, et un mélange de ceux-ci.

8. Composition conforme à l'une des revendications 1 à 7, dans laquelle la phase huileuse comprend au moins une huile qui n'est pas une huile de silicone.

9. Composition conforme à l'une des revendications 1 à 8, dans laquelle la proportion dudit ester (A) d'acide N-(acyle à longue chaîne)-aminé neutre vaut de 0,01 à 20 %, en poids rapporté au poids total de la composition.

10. Composition conforme à l'une des revendications 1 à 9, dans laquelle la proportion dudit polyol (B) comportant trois groupes hydroxyle ou plus vaut de 0,01 à 30 %, en poids rapporté au poids total de la composition.

11. Composition conforme à l'une des revendications 1 à 10, dans laquelle l'émulsion est une émulsion de type huile-dans-eau.

12. Composition conforme à l'une des revendications 1 à 11, laquelle composition est conçue pour l'hydratation de la peau.

13. Procédé cosmétique de traitement d'une peau sèche et/ou de lèvres sèches, comprenant l'application topique d'une composition conforme à l'une des revendications 1 à 11 à ladite peau sèche et/ou auxdites lèvres sèches.

14. Procédé cosmétique d'hydratation de la peau et/ou des lèvres, comprenant l'application topique d'une composition conforme à l'une des revendications 1 à 11 à ladite peau et/ou auxdites lèvres.
